# EUROPEAN PATENT APPLICATION

(11) **EP 2 976 997 A1**
(43) Date of publication of application: **27.01.2016**
(21) Application number: 15180580.1
(22) Date of filing: 03.05.2013
(51) Int. Cl.: A61B 5/00

(54) **ESTIMATING BODY FAT IN A USER**

(30) Priority: 03.05.2012 US 201261642226 P; 02.05.2013 US 201313886173
(62) Divisional of application: 13784171.4
(71) Applicant: Aliphcom, Inc., San Francisco, CA 94103 (US); Raskin, Aza, San Francisco, California 94107 (US)
(72) Inventor: RASKIN, Aza, San Francisco, CA 94107 (US)
(74) Representative: Johnson, Richard Alan

(57) **Abstract**

Embodiments of the present application relate generally to electrical and electronic hardware, computer software, wired and wireless network communications, wearable, hand held, portable computing devices for facilitating communication of information, and the fields of healthcare and personal health. More specifically the present application relates to a new and useful systems, methods and apparatus for estimating body fat in a user with applications in the healthcare and personal health fields. An electronic device, such as a portable electronic device (e.g., smartphone, pad, tablet, etc.) may include software (e.g., an APP) to implement body fat estimates of a user and may use hardware and/or software resident in the electronic device (e.g., display, accelerometer, gyroscopes, transducers, vibration engines, speakers, microphones, GPS capability, etc.) to aid a user in placing the electronic device at instructed location on the user's body and to apply an impulse to the body at instructed locations.

## Description

### FIELD

Embodiments of the present application relate generally to electrical and electronic hardware, computer software, wired and wireless network communications, wearable, hand held, portable computing devices for facilitating communication of information, and the fields of healthcare and personal health. More specifically the present application relates to a new and useful systems, methods and apparatus for estimating body fat in a user with applications in the healthcare and personal health fields.

### BACKGROUND

Obesity and extensive excess body fat is reaching epidemic proportions in the United States and developing countries worldwide. Consumer-grade scales are used by many individuals to track weight fluctuations. Though many correlate body weight with health, body weight may not always be an accurate health indicator. Rather, body fat can more closely correlate with the individual health and health risks, such as heart disease and diabetes. However, body fat measurement equipment (e.g., the type used by medical professionals) is typically expensive, specialized, difficult to use, and is rarely suited to a consumer environment.

Thus, there exists a need in the personal health and healthcare fields to create new and useful systems, methods and apparatus for estimating body fat in a user.

### BRIEF DESCRIPTION OF THE DRAWINGS

Various embodiments or examples ("examples") of the invention are disclosed in the following detailed description and the accompanying drawings. The drawings are not necessarily to scale:
FIG. 1A depicts one example of a flow diagram for estimating body fat of a user according to an embodiment of the present application;
FIG. 1B depicts another example of a flow diagram for estimating body fat of a user according to an embodiment of the present application;
FIG. 3 depicts a graphical representation of an output according to an embodiment of the present application;
FIG. 4A depicts a graphical representation of an output according to an embodiment of the present application;
FIG. 4B depicts one example of a graphical representation instructing a user according to an embodiment of the present application;
FIG. 4C depicts another example of a graphical representation instructing a user according to an embodiment of the present application;
FIG. 5 depicts one example of a graph of a plurality of distinct time-domain channels according to an embodiment of the present application;
FIG. 6 depicts one example of a graph of frequency-domain power spectral density signals according to an embodiment of the present application;
FIGS. 7A - 7B depict various examples of body shapes according to an embodiment of the present application;
FIGS. 8A - 8B depict examples of positioning an electronic device on a body of a user according to an embodiment of the present application;
FIG. 9 depicts one example of a system including two wirelessly enabled user devices for estimating body fat in a user according to an embodiment of the present application; and
FIG. 10 depicts an example of a plurality of user devices for estimating body fat of a user according to an embodiment of the present application.

### DETAILED DESCRIPTION

Various embodiments or examples may be implemented in numerous ways, including as a system, a process, an apparatus, a user interface, or a series of program instructions on a non-transitory computer readable medium such as a computer readable storage medium or a computer network where the program instructions are sent over optical, electronic, or wireless communication links. In general, operations of disclosed processes may be performed in an arbitrary order, unless otherwise provided in the claims.

A detailed description of one or more examples is provided below along with accompanying figures. The detailed description is provided in connection with such examples, but is not limited to any particular example. The scope is limited only by the claims and numerous alternatives, modifications, and equivalents are encompassed. Numerous specific details are set forth in the following description in order to provide a thorough understanding. These details are provided for the purpose of example and the described techniques may be practiced according to the claims without some or all of these specific details. For clarity, technical material that is known in the technical fields related to the examples has not been described in detail to avoid unnecessarily obscuring the description.

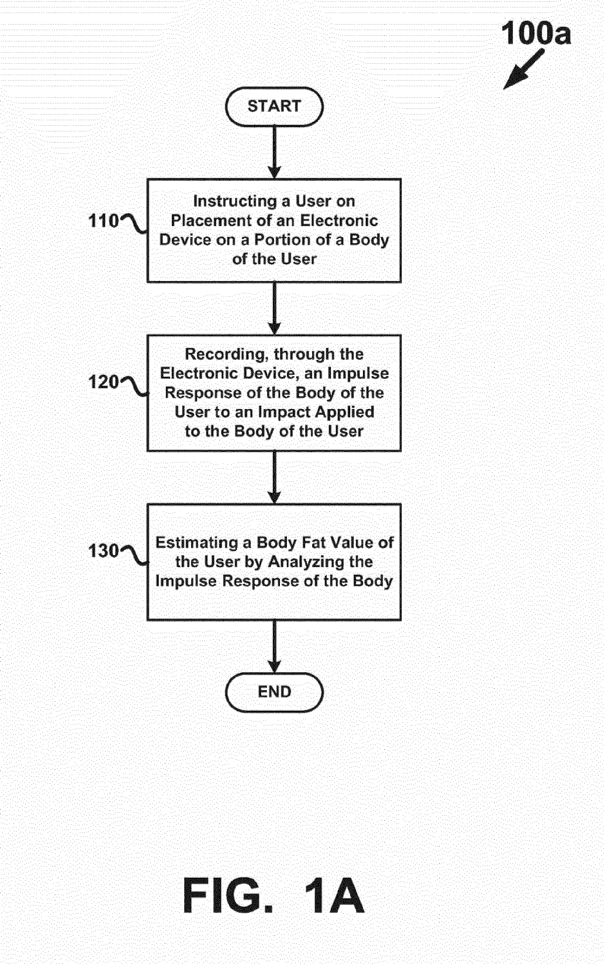

FIG. 1A depicts one example of a flow diagram 100a for estimating body fat of a user. As depicted in FIG. 1A, flow diagram 100a (flow 100a hereinafter) for estimating body fat in a user may include but is not limited to: instructing a user on placement of an electronic device on a portion of a body of the user at a stage 110; recording, through the electronic device, an impulse response of the body of the user to an impact applied to the body of the user at a stage 120; and estimating a body fat value of the user by analyzing the impulse response of the body at a stage 130.

The stages of flow 100a may be implemented as an application (e.g., a non-transitory computer readable medium) executing on an electronic device which may estimate the body fat value of the user through a form of plessimetry, wherein analysis of the bodily impulse response to the bodily impact informs the body fat value. The impact on the body may be generated by the electronic device itself or by some other instrumentality. For example, the impact may alternatively be generated by the user or a second individual or device. The body fat value can be any one or more of body mass index, body fat percentage, body fat volume, body fat mass, or any other suitable metric.

The electronic device may be a mobile handheld electronic device commonly carried by the user including but not limited to: a data capable strap band, wristband, wristwatch, digital watch, or wireless activity monitoring and reporting device; a smartphone; cellular phone; tablet; tablet computer; pad device (e.g., an iPad); touch screen device; touch screen computer; laptop computer; personal computer; server; personal digital assistant (PDA); portable gaming device; a mobile electronic device; and a wireless media device. The electronic device may include a buzzer, vibrator, or other electromechanical actuator capable of generating the impact (e.g., a mechanical force or vibration for the impulse) and an accelerometer or other sensor capable of capturing linear or rotational motion and/or accelerations. The electronic device may include additional sensors, such as a gyroscope, a camera, an image sensor, a proximity sensor, and a user input region, such as a keyboard or touch screen, that may increase the functionality of the flow 100a and/or improve the accuracy of the estimate body fat value. The electronic device also may include a display that visually depicts to the user the placement instruction of stage 110, though the display may depict any other instruction or user metric. Alternatively, the instruction of stage 110 may be provided to the user through a speaker, headphones, earphones, wireless headset, or other output mechanism of the electronic device.

As another example, flow 100a may be implemented as a native application executing on a mobile electronic device. In this example, the native application may preferably execute on a variety of mobile electronic devices of a variety of types, forms, and/or operating systems. Furthermore, in this example, the native application may preferably execute on preexisting, non-specialized mobile electronic devices such that a vast number of users may access the flow 100a to estimate a body fat value without purchasing additional or specialized hardware. However, portions of the flow 100a may alternatively be implemented on a remote server or other form of compute engine, network, the Cloud, the Internet, or separate electronic device. Furthermore, portions of the flow 100a, such as recordation of the impulse response at stage 120, may be implemented by an electronic device that is any of a specialized or standalone body fat value-specific device, a peripheral device for a laptop computer, desktop computer, or other processing device, or any other suitable device.

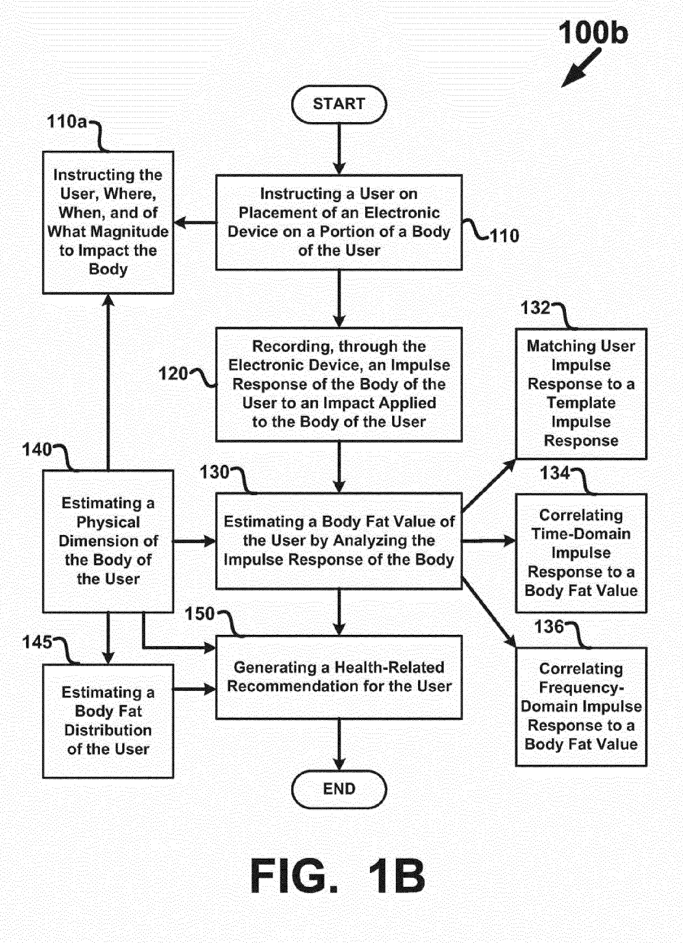

As depicted in FIGS. 1A and 1B, the stage 110 of the flow 100a describes instructing placement of an electronic device on a portion of a body of the user. In a variation of the flow 100a in which the user generates the impact, stage 110 may further include instructing the user where and when to generate the impact on the body. In one example implementation, the user is instructed to strike (i.e. impact) the electronic device that is held against the portion of the body of the user. In another example implementation, the user is instructed to hold the electronic device against one portion of the body and to strike a second portion of the body. In yet another example implementation, the user is instructed to hold the electronic device against one portion of the body and to strike two or more different portions of the body.

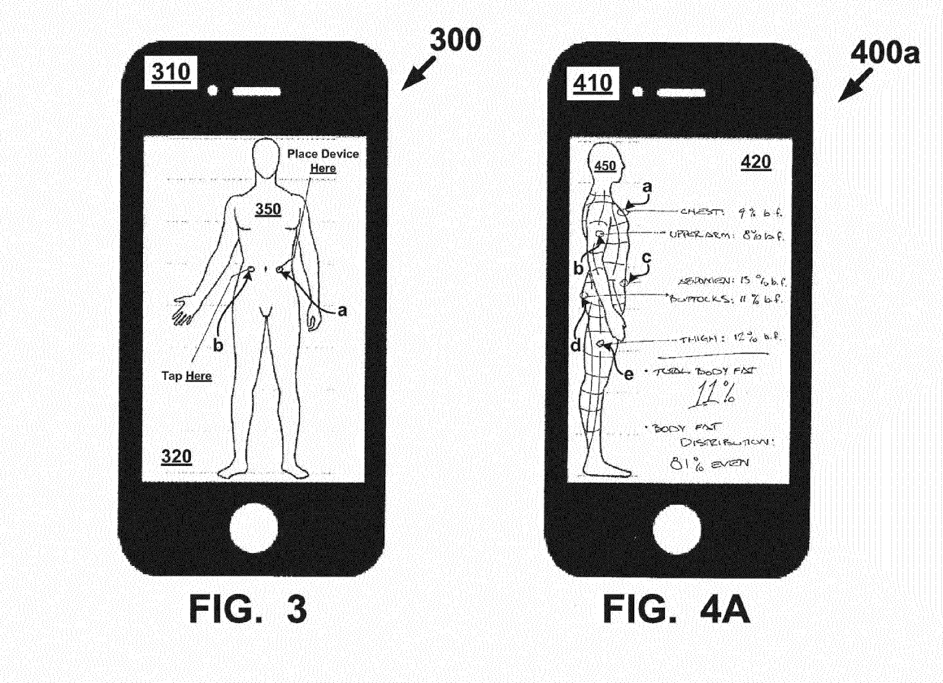

The instructions at stage 110 may preferably be rendered on a digital display 320 (e.g., a LCD, touch screen, or the like, such as depicted in FIGS. 3 - 4C) of an electronic device 310. In FIG. 3, the instructions may preferably include a digital rendering 300 of a body 350 including a desired placement, denoted as "a", of the electronic device 310 against the body 350. In the examples in which the user generates the impact, the rendering may further include one or more preferred impact locations denoted as "b". However, placement of the electronic device 310 and/or location(s) "a" of the impact(s) "b" may be communicated in any other way, such as with text-based instruction rendered on the display 320, audio-based instruction played through a speaker, headphone, earphones, wireless headset, or tactile feedback generated by a vibrator or other electromechanical actuator carried by the device 310.

Placement of the electronic device for body fat value readings may be specified by a placement schedule that includes one or more specific locations on the body. For example, the placement schedule may include left, right and center of the abdomen, inner and outer right or left thigh, upper right and left chest, and inner and outer left or right upper arm. In FIG. 4A, display 420 of electronic device 410 includes a profile view of a body 450 with five different locations a - e for placement of the electronic device 410 for taking body fat readings. After instructions for those locations are executed by the user of device 410, the output for the body fat values may be displayed on display 420 along with, or without, the profile view of the body 450. Other locations of the body may additionally or alternatively be included in the placement schedule. Furthermore, bodily symmetry across the body center line may also be assumed such that readings are not necessary on both the left and the right side of the user. In other examples, bodily symmetry need not be assumed and the readings may be taken on both sides of the body only or in some other non-symmetrical pattern.

In a variation of the electronic device that includes an accelerometer, the flow 100a may implement dead reckoning to guide user placement of the electronic device on one or more portions of the body of the user. In one example implementation in which the accelerometer comprises a three-axis accelerometer, the user is instructed to place or hold the electronic device in a reference location and the reference location is preferably subsequently recorded. The reference location may be centered between the feet of the user when the user is standing, in a hand of the user with the arms of the user relaxed at his side, on a table or desk of standard height (e.g., ∼30" tall), or any other suitable reference location. In this example implementation, subsequent positions of the electronic device are determined by integrating accelerations measured by the accelerometer (i.e. dead reckoning), and user placement of the electronic device is guided according to an estimated distance from the reference location. In one example, the user is instructed to place the electronic device every four inches up the torso, beginning at the waistline, wherein stage 110 indicates the proper distance between readings by estimating vertical distance changes through dead reckoning. In another example, the accelerometer additionally or alternatively informs horizontal placement of the electronic device, such as evenly spaced around the abdomen, a thigh, or the buttocks. In a further example, the height of the user is estimated by integrating accelerations measured by the accelerometer as the user moves the electronic device from head to foot (or vice versa), and preferred reading locations are then determined based upon the height of the user, such as predominantly around the abdomen and thighs.

In a variation of the electronic device that includes an accelerometer and a gyroscope, the flow 100a may implement inertial navigation to guide user placement of the electronic device on one or more portions of the body of the user, such as similar to dead reckoning as described above. In this variation, the accelerometer may preferably be a three-axis accelerometer and the gyroscope may preferably be a three-axis gyroscope. However, the data from the accelerometer and/or gyroscope of the electronic device may be used in any other way to inform or guide the impact location and/or placement of the electronic device on the body of the user.

As depicted in FIGS. 1A and 1B, stage 120 of the flow 100a describes recording, through the electronic device, an impulse response of the body of the user to an impact applied to the body of the user. In the variation of the flow 100a described above, the impact is generated by the user, either on the electronic device or on a second portion of the body. In another variation of the flow 100a, the impact is generated by a vibrator or other electromechanical actuator incorporated in the electronic device. The impact may preferably be an impulse that decays over time as the impulse is absorbed by the body of the user. The impulse and subsequent decay (i.e. impulse response) through the body may preferably be captured by the accelerometer as a time-domain amplitude output of linear accelerations along one or more axes. The accelerometer output that includes the impulse response may preferably be stored digitally and locally on the electronic device (e.g., in a data storage system such as Flash memory). However, the accelerometer data may be stored in any other way and on any other device, component, or subcomponent. Additionally or alternatively, in the variation of the electronic device that includes a gyroscope, the impulse response may be captured by the gyroscope as a time-domain amplitude output of rotational accelerations around one or more axes.

In the variation of the flow 100a in which the electronic device generates the impact, the impact may preferably be a vibration of known amplitude, frequency, and duration. The impact may further include a series of vibrations of the same or different amplitude, frequency, and/or duration. The amplitude, frequency, and/or duration of the vibration(s) may also be dependent on the portion of the body that is undergoing testing. For example, the frequency may be lower and the duration greater for the impact that is generated on the abdomen than for an impact that is generated on the thigh or upper arm. In this variation, the impulse may be initiated according to a timer, initiated upon a user input indicating that the electronic device is properly positioned, initiated when a proximity sensor or camera in the electronic device determines that the electronic device is arranged against the body of the user, or initiated upon any other event, input, or sensor output.

In the variation of the flow 100a in which the user generates the impact, the electronic device may be armed for impulse response recordation according to a variety of schema. In a first example implementation, once the native application executing the flow 100a is opened by the user on the electronic device (e.g., launching an APP on a smartphone or tablet/pad), the application is armed for recordation. In this example implementation, a high-amplitude signal output from the accelerometer, such as a measured acceleration greater than a threshold acceleration, may then initiate recordation of the impulse response. In another example implementation in which the electronic device includes a proximity sensor, recordation of the accelerometer output signal is armed or triggered when the electronic device is determined to be adjacent the body of the user. A further example implementation includes alerting the user, such as visually, audibly or tactilely, to percuss the body (e.g., to apply a tap the body), wherein recordation of the accelerometer output is armed or triggered when the user is alerted. However, recordation of the impulse response in stage 120 may be recorded according to any schema or through any other device, component, or subcomponent. For example, the electronic device may include a communications link to an external system and transmit the impulse response to the external system over the communications link. The communications link may be wired (e.g., Ethernet or LAN) and/or wireless (e.g., WiFi, Cellular, Bluetooth®, etc.).

As depicted in FIGS. 1A and 1B, stage 130 of the flow 100a describes estimating a body fat value of the user by analyzing the impulse response of the body. Stage 130 may include generating the body fat value based upon a single recorded impulse response at a single body portion, based upon a several recorded impulse responses proximal a single body portion, based upon a several recorded impulse responses, each proximal a different body portion, or based upon a several recorded impulse responses including multiple impulse response proximal a various body portions.

In stage 130, the body fat value of the user may preferably be estimated by aggregating separate estimated fat values for different portions of the user's body. For example, an estimated fat value of the lower torso may preferably be combined with distinct estimated fat values of the upper torso, the left upper arm, the right upper arm, the left thigh, the right thigh, and the neck. Each distinct fat value for each portion of the body may preferably be estimated according to a separate impulse response analysis for each of the portions. The distinct fat values may then be aggregated or combined, such as with a weighted average or other schema, to generate a global estimated body fat value for the user, as depicted in FIG. 4A. Alternatively, the body fat value may remain localized to a particular portion of the user's body, such as the abdomen or lower torso. However, the body fat value may include any other one or more portions of the body of the user.

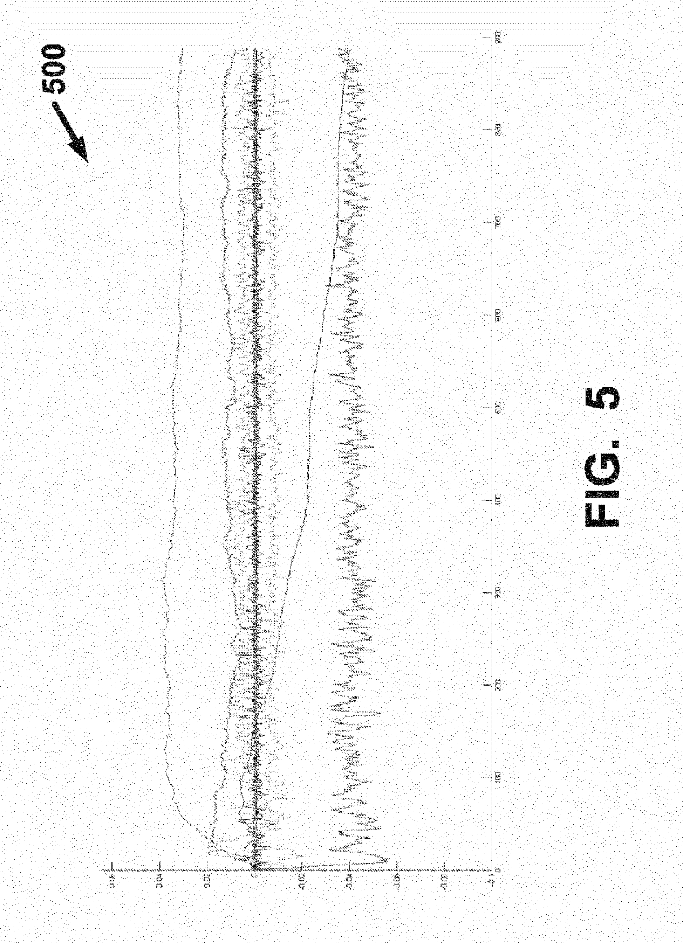

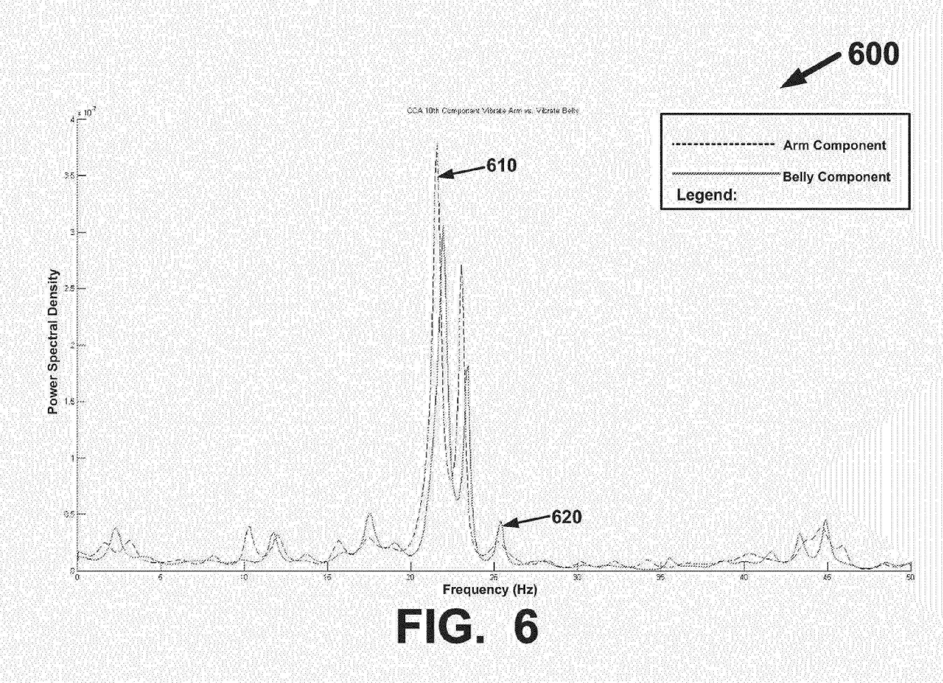

In one variation of the flow 100a in which the electronic device incorporates a three-axis accelerometer and a three-axis gyroscope: three linear accelerations; three rotational accelerations; three linear velocities; and three rotational velocities are aggregated into a set of twelve distinct time-domain channels, as depicted in graph 500 of FIG. 5. The frequency-domain power spectral density signal may then be extracted from the time domain channels through a Fourier transform or similar method. As depicted in graph 600 of FIG. 6, certain frequencies or frequency ranges may correlate with particular portions of the body, such as the abdomen or upper arm, and amplitude peaks at certain frequencies or within certain frequency ranges may correspond with fat values for particular portions of the body. In graph 600 the dashed line 610 represent an arm component and solid line 620 represents a belly component as depicted in the legend of FIG. 6. In this variation, a single transformed channel may be correlated with the body fat value, or a combination of several transformed channels may be combined or averaged to calculate the body fat value. However, any other number of channels from any other type of sensor may be manipulated in stage 130 according to this variation. Stages 134 and/or 136 of flow 100b may be used individually or in conjunction with stage 130 to correlate time-domain and/or frequency-domain impulse response to a body fat value.

Furthermore, in this variation, one or more particular frequencies or frequency bands may be associated with a particular type of fat, including but not limited to: subcutaneous fat; intramuscular fat; yellow bone marrow; adipose breast tissue; or visceral fat including mesenteric, epididymal white adipose tissue, and pararenal depots. In this variation, the amplitude of a particular frequency in a frequency-domain plot of the impulse response of the body at some time after the impact (i.e., after the impulse is applied to the user's body), as measured by the accelerometer and/or gyroscope, may be correlated with a mass, volume, or density of a particular type of fat or fat depot in the portion of the body. Finally, the correlation between power density of a particular frequency and a body fat value may be linear, exponential, logarithmic, or of any other relationship and may preferably be determined experimentally and augmented through machine learning.

Generally, the resonant frequency of a portion of the body may be indicative of the body fat value of that the portion of the body. In this variation, the peak frequency in the frequency-domain signal of one or more channels may correlate with the resonant frequency of the portion of the body and thus correspond with the body fat value. Additionally or alternatively, the resonant frequency of the electronic device may be accounted for and/or removed from recorded impulse response signals of the body of the user, such as with a bandpass filter or the like.

In another variation of the flow 100a, the amount of time that passes from the initial impulse to substantial decay of the impulse, such as 95% of the acceleration amplitude at impulse, may be indicative of the body fat value of the user. In this variation, the time-domain impulse response of the body of the user substantially directly indicates the body fat value. For example, an impulse that is measured at the center of the abdomen as ∼1 g (i.e. ∼9.8m/S1) acceleration and that decays to less than .05g over 4.2 seconds may indicate a body fat percentage proximal the abdomen to be ∼18%; whereas, an impulse that is measured at the center of the abdomen as ∼1g (i.e. ∼9.8m/S1) acceleration and that decays to less than .05g over 1.1 second may indicate a body fat percentage proximal the abdomen to be ∼7.5%. However, decay of the impulse response over time may correlate with body fat value in any other way and is not limited to the above examples.

In yet another variation of the flow 100a, the recorded impulse response of the user may be matched with a template impulse response in a library of template impulse responses of template users. In this variation, each template impulse response may be associated with a known body fat value. Each template impulse response may be further associated with any one or more of an age, gender, medical history, medication, diet, diet plan, water consumption, exercise regimen, average activity level, physical body dimension, physical condition, body weight, placement of impact and/or electronic device on the body, or other relevant characteristic of a template user or body fat measurement technique. In one example implementation of this variation, the time-domain impulse response of the user may be compared substantially directly with time-domain template impulse responses of template users, wherein the time-dependent decay of the initial impact, through the portion of the body of the user, may be matched to a template time-dependent decay of an impact on the body of the template user of known body fat value. In another example implementation of this variation, the frequency-domain impulse response of the user may be compared substantially directly with frequency-domain template impulse responses of template users, wherein frequency-domain power spectral density data of the impulse response may be matched to template frequency-domain power spectral density data of a template user of known body fat value. In the foregoing example implementations, comparison of the user and template user impulse responses may preferably account for any one or more of age, gender, medical history, medication, diet, diet plan, recent food or water consumption, exercise regimen, average activity level, physical body dimension, physical condition, and/or body weight of the user and template user. However, the impulse response of the user may be compared to and matched with a template impulse response of a template user in any other way or according to any other schema. Furthermore, the impulse response of the user may be manipulated and analyzed in any other way to estimate the body fat value of the user.

The foregoing variations may preferably implement machine learning, wherein new impulse response data sets are added to historic impulse response data sets to further teach a correlation between body fat and body impulse response. Stage 130 may preferably implement supervised machine learning, wherein stage 130 accesses a set of training data that includes template impulse responses labeled with known body fat values. A learning procedure may then transform the training data into generalized patterns to create a model for subsequent analysis of new impulse response data particular to the user. However, stage 130 may alternatively implement unsupervised machine learning (e.g., clustering) or semi-supervised machine learning, wherein all or at least some of the training data is not labeled, respectively. Stage 130 may further implement feature extraction, canonical correlation analysis (CCA), principal component analysis (PCA), piecewise linear discriminant analysis (pLDA), feature selection, or any other suitable statistical or data analysis technique to isolate relevant impulse response features and/or prune redundant or irrelevant information from the impulse response.

Machine learning may comprise the electronic device and associated algorithms executed on one or more processors of the electronic device to implement the machine learning internal to the electronic device. Training data and/or the templates may be resident in data storage in the electronic device or may be obtained in whole or in part from an external source using a wired and/or wireless communications link. External sources may include but are not limited to the Internet, the Cloud, a network, a server, a personal computer or laptop computer, network attached storage (NAS), RAID storage, Flash memory stick or card, non-volatile memory, disk memory (e.g., optical or magnetic), a web site or web page, a wired and/or wirelessly connected device that provides some or all of the training data and/or the templates, just to name a few. As one example, the user may be wearing a wireless device such as a data capable strap band, wristband, wristwatch, digital watch, or wireless activity monitoring and reporting device that includes some or all of the training data and/or the templates and the user's electronic device and the wireless device establish a wireless communications link with each other to allow access to and/or modification of the training data and/or the templates.

Furthermore, any of the foregoing variations may account for any one or more of the age, gender, medical history or condition (e.g., pneumothorax), medication, diet, diet plan, recent food or liquid consumption, exercise regimen, average activity level, physical body dimension, weight, placement of impact and/or electronic device on the body, or other relevant characteristic of a user or body fat measurement technique. Relevant user data may be determined experimentally, such as through body dimension measurement techniques described below or by communicating with a digital scale used by the user. Additionally or alternatively, relevant user data may be mined from existing user data, such as from a social (e.g., Facebook®), professional (Linkedln®), a health or dietary profile of the user (e.g., The Eatery), or a user medical record. However, the user may supply any of this information manually. Relevant user data may be obtained from a wireless device such as a data capable strap band, wristband, wristwatch, digital watch, or wireless activity monitoring and reporting device that the user may wear or have access to, and the relevant user data and optionally other biometric data gathered from the user may be communicated (e.g., wirelessly) to the electronic device.

In FIG. 1B, one variation of the flow 100a is denoted as flow 100b and includes a stage 140, which describes estimating a physical dimension of the body of the user. The physical dimension of the user may be any of height, inseam, wingspan, or width, depth, or diameter at the neck, chest, upper arm, navel, waist, buttocks, or thigh, or any other suitable bodily dimension. The physical dimension may additionally or alternatively be a bodily contour, such as curvature around the abdomen, hips, thighs, buttocks or a general body shape. In the variation depicted in flow 100b, the physical dimension of the user may indicate distribution of fat throughout the body of the user, may augment or verify the estimated body fat value of stage 130, and/or may inform a health recommendation made to the user.

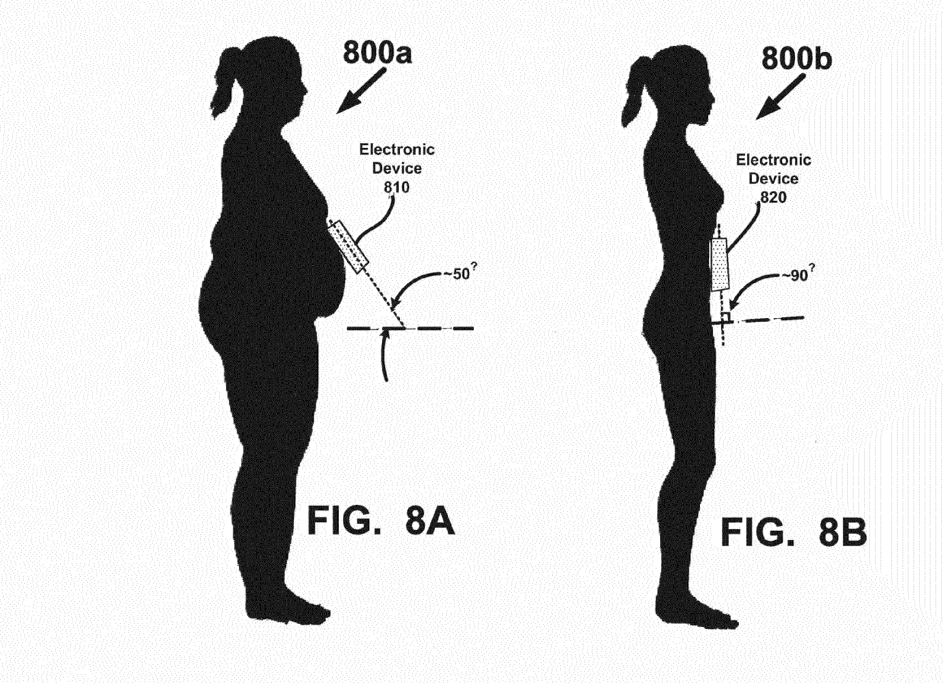

In one example implementation, while held against the portion of the body of the user, the orientation of the electronic device may be estimated by analyzing the accelerometer output, wherein the orientation indicates a contour of the body of the user. The orientation of the electronic device may be determined prior to, during, or following recordation of the impulse response of the body of the user in stage 120 or at any other suitable time. In one example, stage 110 includes instructing the user to hold the electronic device centered horizontally on the body just below the Xiphoid Process (or sternum), and stage 140 may include estimating the fore-aft angle of the electronic device, held adjacent the body of the user, just before the impact. In this example, the angle of the electronic device 820 to the ground that is substantially vertical (e.g., ∼90°) indicates that the user has minimal visceral (i.e. abdominal) fat, as depicted in FIG. 8B, wherein the angle of the electronic device 810 to the ground that is substantially acute (e.g., ∼50°) indicates that the user has substantially visceral fat, as depicted in FIG. 8A. In one variation of the flow 100a, the user may be instructed to retain the electronic device against substantially the same portion of the body for each reading in order to minimize errors or outliers in the estimated body fat value and/or the estimated body contour. In this variation, trends in the orientation of the electronic device over time may also indicate changes in user weight. In another variation of the flow 100b, the user may be instructed to retain the electronic device against different portions of the body for each subsequent reading such that a contour map of the body of the user may be created. In the latter variation, the position of the electronic device on the body of the user may be determined, such as relative a reference point, as described above, or the position of the electronic device on the user may be estimated based upon the preferred placement suggested to the user in stage 110. The above variations relating to more detailed instructions to the user associated with the instructing at the stage 110 are denoted in FIG. 1B as a stage 110a which discloses instructing the user, where, when, and of what magnitude to impact the body.

In another example implementation and as described above, the flow 100a may implement dead reckoning or inertial navigation to estimate a physical dimension of the body of the user that is a linear distance. In one example and as described above, user height may be determined through dead reckoning or inertial navigation as the user moves the electronic device from proximal the feet to or from proximal the top of the head. In another example, a width dimension at the waist may be calculated through dead reckoning or inertial navigation as the user transfers the electronic device from adjacent one hip to adjacent the other. In a further example, a depth dimension at the navel may be estimated as the user transfers the electronic device from adjacent the back to adjacent the navel. The instructing disclosed in stage 110a may be used to guide the user as to correct placement of the electronic device on the user's body and application of the impact force necessary to estimate the physical dimensions of the user's body.

In a further example implementation, the flow 100a may additionally or alternatively implement dead reckoning or inertial navigation to estimate a physical dimension of the body of the user that is an external body contour. In this variation, the contour of the body of the user may be correlated with estimated positions of the electronic device as the user moves the electronic device along an external portion of the body. In a first example, the user may drag the electronic device along the body from proximal the right ankle, along the front of the shin, over the knee and front thigh, across the hip, and over the center of the abdomen and chest, terminating at the center of the collarbone, wherein the path is recorded through dead reckoning or inertial navigation to generate a substantially vertical contour line of the body of the user. In this example, the contour line may capture depth changes or deviations from an imaginary vertical plane, wherein a large deviation from the imaginary vertical plane proximal the waist or abdomen may indicate a large amount of visceral fat. In a second example, the user may drag the electronic device from the back left flank, around to the front of the abdomen, and over the navel, terminating in the back right flank, wherein the path is recorded through dead reckoning or inertial navigation to generate a substantially horizontal contour line proximal the waist of the user. In a third example that combines the foregoing first and second examples, the user passes the electronic device vertically and horizontally along various portions of the body, wherein the various substantially horizontal and vertical paths are recorded through dead reckoning or inertial navigation and assembled to generate a three-dimensional contour plot of the body of the user, as depicted in FIG. 4A. In FIG. 4A, a graphical representation 400a of an output is presented on a display 420 of a user device 410 (e.g., a smartphone) and may include a three-dimensional contour plot of the user's body. The profile view depicted on display 420 may include information and/or results for various portions a - e of the user's body that were analyzed by user device 410 (e.g., as per the instructing of stages 110 and/or 110a). This three-dimensional contour plot of the body of the user may suggest body shape, body proportions, fitness level, distribution of muscle and fat, or any other relevant physical metric or dimension of the user. Moreover, the instructing at stage 110a may be used to guide the user as to correct placement of the electronic device on the user's body and application of the impact force necessary to estimate the physical dimensions of the user's body.

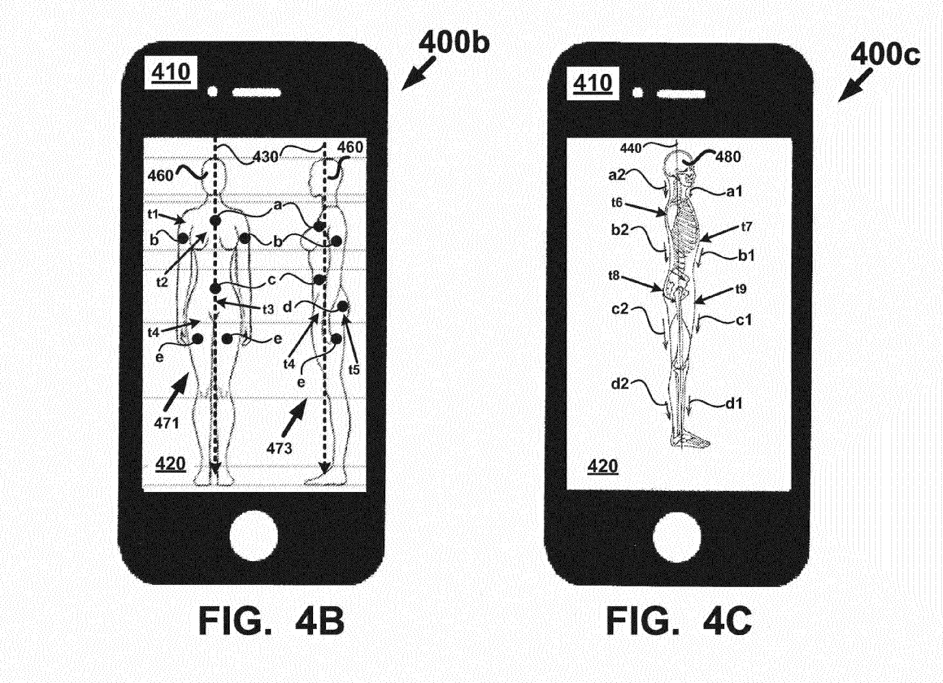

To aid the user in following the correct path and positioning of the user device on the body, an example body contour with paths and positions along the body contour for positioning the electronic device and impulse forces to be applied at those positions may be presented on the display 420 of the devices depicted in FIGS. 4B and 4C, and after the user has traced the path suggested by the example body contour, the actual output results for the user body contour may then be displayed by the device as described above in reference to FIG. 4A, graphically displaying output results including but not limited to percent body fat for: chest; upper arm; abdomen; buttocks; thigh; total body fat percentage (e.g., 11 %); body fat distribution, etc.

In FIG. 4B, a graphical representation 400b includes front 471 and profile 473 views of a body 460 presented on a display 420 of electronic device 410. Here, positions/locations a - e along a contour of the body 460 are instructed (e.g., at stages 110 and/or 110a) to the user. Here, the path and locations a - e along the path may or may not be symmetrical with a center line of symmetry 430 of body 460. The instructions depicted in graphical representation 400b may include one or more impact points t1 - t5 where impulses (e.g., at the stage 120) are to be applied by the user or some other instrumentation. The user may or may not position the electronic device 410 at all of the locations a - e as instructed and may or may not apply the impulses at all of the impact locations t1 - t5. Along the center line of symmetry 430 the flows 100a and/or 100b may take into count the handedness of the user such that for a left handed user, points b and e on that user's right side may be where the user positions the electronic device 410 with the user's left hand and applies the impulses with the right hand. Conversely, for a right handed user, points b and e on that user's left side may be where the user positions the electronic device 410 with the user's right hand and applies the impulses with the left hand. Profile view 473 depicts a position d on the buttocks and an impact point t5 which are not depicted in the front view 471. Along the center line of symmetry 430 the flows 100a and/or 100b may include instructions for the user to use the electronic device 410 to take a height estimate by positioning the electronic device 410 at the top of the head of the user and moving along the center line of symmetry 430 to the user's feet or toes.

In FIG 4C, a graphical representation 400c includes a profile view of a body 480 having a center line of symmetry 440 displayed on a screen 420 of electronic device 410. Along a front of the body 480, display 420 may include one or more points along a contour path denoted by arrows a1 - d1 at which to position the electronic device 410 and one or more locations to apply an impulse denoted as t7 and t9. The user who wants the body fat estimate may or may not be able to perform the instructions (e.g., from the stages 110 and/or 110a). To that end another person such as a friend, family member, health care professional, care taker or the like may assist the user by implementing the instructions on the user's behalf. As one example, in FIG. 4C, the desired contour and/or path may be on the user's backside as denoted by arrows a2 -d2 and one or more impact point's t6 and t8 may also be on the user's backside. Accordingly, a helper may assist the user by reviewing the instructions on the graphical representation 400c and stand behind the user and implement the instructions, such as following the path, placing the electronic device 410 at instructed positions along the path, and applying the impacts at the instructed points on the body of the user. Although FIGS. 3-4C depict a user's body in a standing position, the user's body need not be in a standing position and the user may be positioned in a prone or supine position on a table, bed, floor, surface or the like and instructions for placement of the device and application of the impulse may be tailored for prone or supine position and displayed for the user in a manner similar to the depictions in FIGS. 3 - 4C. In some examples, the user may be in a seated position or some other position, so long as those positions are appropriate for obtaining accurate results using the user device.

In another example implementation in which the electronic device that includes a camera or other imaging system, the user may be instructed (e.g., at the stage 110a) to take a full-body image of himself, such as while standing before a mirror and preferably while wearing minimal clothing or minimal loose-fitting clothing. In this example implementation, stage 140 may include analyzing the image of the user to determine a general shape, dimension, or contour of the user based upon a reference dimension, such as a user height, wingspan, inseam, or hip width dimension. The reference dimension may be entered manually by the user or determined experimentally, such as in any of the foregoing example implementations. Alternatively, dimensions of the user may be estimated in stage 140 by accessing focus data of the camera when the image is taken. The image may be either or both of a front view and/or a side view of the user. In this example implementation, the silhouette of the user may preferably be isolated from a background and an overall body shape thus determined. Additionally or alternatively, shadows or other features on the body of the user may inform bodily contours. For example, substantially horizontal shadows around the abdomen of the user in either a front or side profile image of the user may indicate skin folds along the torso, which may correlate with large amount of visceral fat or modify the body fat estimation technique of stage 130.

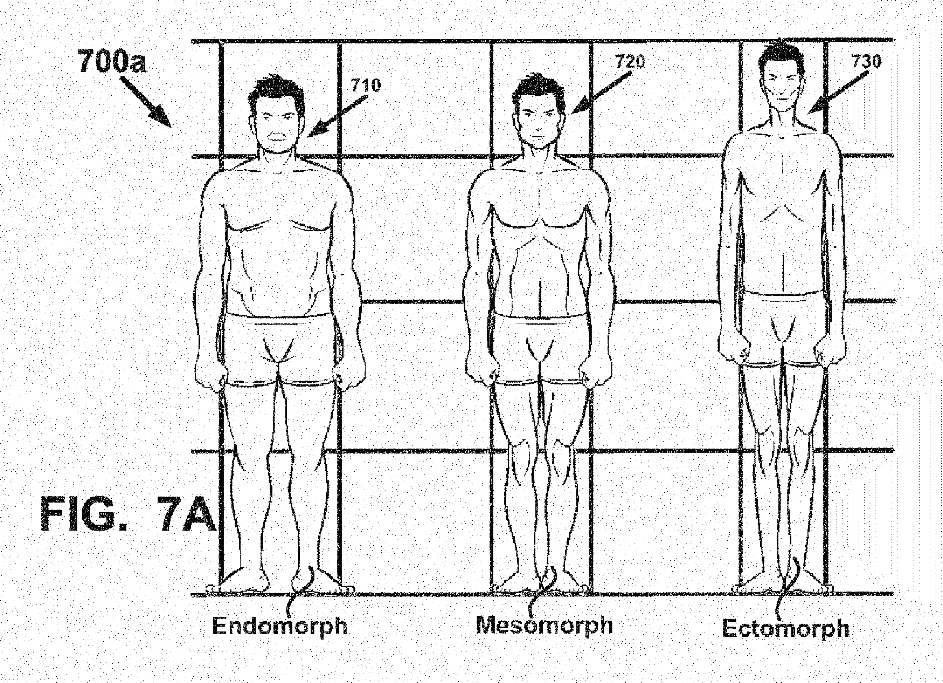

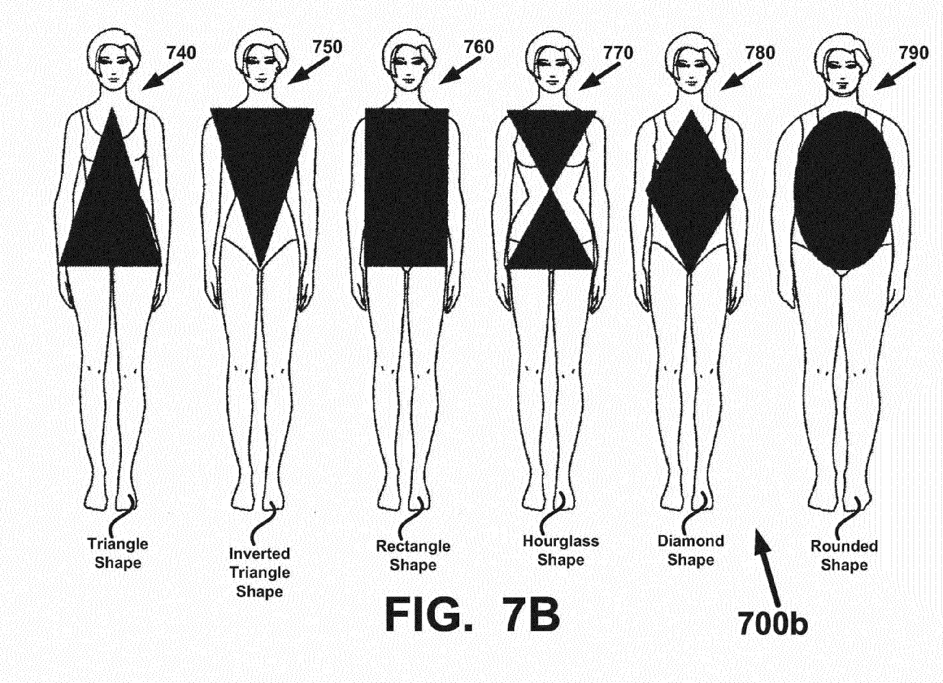

The physical dimension or shape of the body of the user that is estimated in stage 140 may be used in a variety of ways. In one variation, the shape of the user indicates the distribution of fat in the user. In this variation, estimating body fat distribution at a stage 145 of flow 100b may suggest, verify, or reinforce a health risk estimate of the user. For example, for a first user and second with similar and relatively high estimated body fat values proximal the abdomen, wherein the body shape of the first user shows proportional distribution of fat throughout the body and wherein the body shape of the second user shows disproportionate distribution of fat in the lower torso, the health risk of the second user is estimated to be substantially higher than that of the first user. In another variation, the physical dimension informs a preferred placement of the electronic device on the body of the user to avoid skin folds or avoid boney regions. In yet another variation, the physical dimension informs selection of a group of template impulse responses from the template library for comparison with the impulse response of the user in stage 130. For example, the template impulse responses may be selected at a stage 132 of flow 100b from the library for comparison with the user impulse response based upon the estimated body shape of the user, such as triangle, inverted triangle, rectangle, hourglass, diamond, rounded, endomorph, mesomorph, or ectomorph shape, as depicted in FIGS. 7A - 7B, where body shapes 700a and 700b include but are not limited to: endomorph 710; mesomorph 720; ectomorph 730 (depicted in FIG. 7A); triangle 740; inverted triangle 750; rectangle 760; hourglass 770; diamond 780; and rounded 790 (depicted in FIG. 7B). In a further variation, the physical dimension may verify or augment the body fat value estimated in stage 130. For example, the estimated body fat value that is relatively high for the user with a physical dimension in line with a substantially fit user may indicate an error in the original body fat value estimation and thus trigger a new test. However, the physical dimension or shape of the body of the user may be used in any other way to verify and/or augment the estimated body fat value in the flow 100a.

Any of the estimated body fat value, body impulse response, physical dimension, and/or body shape of the user may be added to a health file or profile of the user. The health file or profile may be stored on and/or maintained by the electronic device, a hospital server or network, a medical clinic server or network, a health insurance provider, an exercise or fitness trainer, a dietary management service, the Internet, the Cloud, a web page, a web site, a Flash memory device, a data storage device, or any other suitable device, server, or network or combination thereof.

As depicted in FIG. 1B, flow 100b includes a stage 150, which describes generating a health-related recommendation for the user. This recommendation may preferably be related to the estimated body fat value and may also be related to a physical dimension or shape of the user. Furthermore, this recommendation may account for trends in estimated body fat values, a physical dimension, or a shape of the user over time. For example, a trend that shows that the body fat value of the user is consistently improving may result in a recommendation in stage 150 that includes encouragement to continue the routine.

The recommendation of stage 150 may be for a change to any of diet, hydration, exercise, stress response, sleep, posture, a daily activity, or any other action, characteristic, or input of the user. The recommendation of stage 150 may also or alternatively be a weight, diet, hydration, sleep, stress, or work goal. Furthermore, the recommendation of stage 150 may also or alternatively be to seek expert medical or nutritional advice, though the recommendation may include any other content or be of any other form.

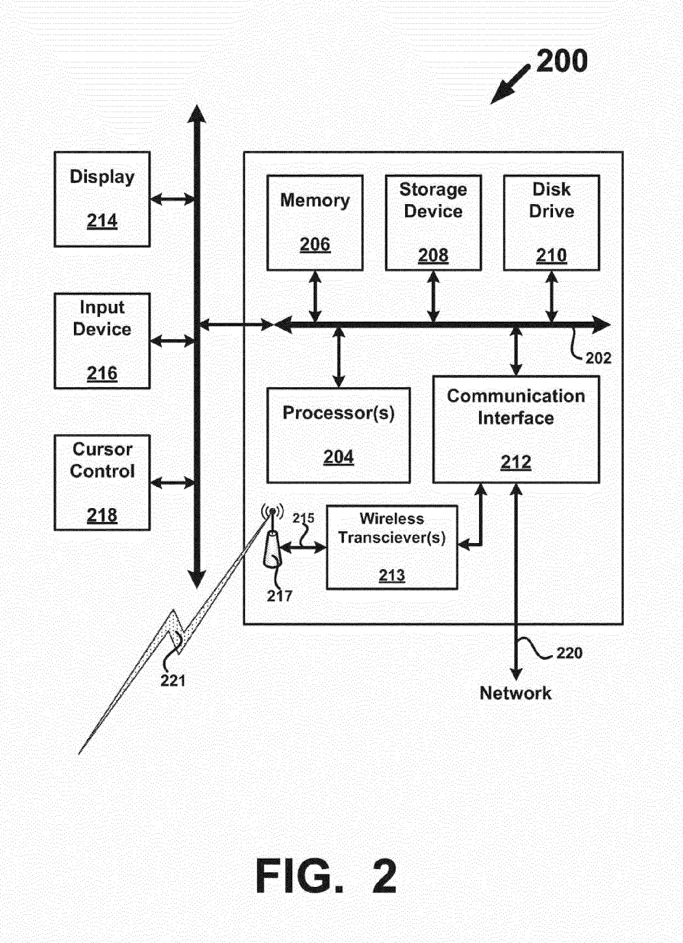

FIG. 2 depicts an exemplary computer system 200 suitable for use in the systems, methods, and apparatus described herein for estimating body fat in a user. In some examples, computer system 200 may be used to implement computer programs, applications, configurations, methods, processes, or other software to perform the above-described techniques. Computer system 200 includes a bus 202 or other communication mechanism for communicating information, which interconnects subsystems and devices, such as one or more processors 204, system memory 206 (e.g., RAM, SRAM, DRAM, Flash), storage device 208 (e.g., Flash, ROM), disk drive 210 (e.g., magnetic, optical, solid state), communication interface 212 (e.g., modem, Ethernet, WiFi), display 214 (e.g., CRT, LCD, touch screen), input device 216 (e.g., keyboard, stylus), and cursor control 218 (e.g., mouse, trackball, stylus). Some of the elements depicted in computer system 200 may be optional, such as elements 214 -218, for example and computer system 200 need not include all of the elements depicted.

According to some examples, computer system 200 performs specific operations by processor 204 executing one or more sequences of one or more instructions stored in system memory 206. Such instructions may be read into system memory 206 from another non-transitory computer readable medium, such as storage device 208 or disk drive 210 (e.g., a HD or SSD). In some examples, circuitry may be used in place of or in combination with software instructions for implementation. The term "non-transitory computer readable medium" refers to any tangible medium that participates in providing instructions to processor 204 for execution. Such a medium may take many forms, including but not limited to, nonvolatile media and volatile media. Non-volatile media includes, for example, optical, magnetic, or solid state disks, such as disk drive 210. Volatile media includes dynamic memory, such as system memory 206. Common forms of non-transitory computer readable media includes, for example, floppy disk, flexible disk, hard disk, SSD, magnetic tape, any other magnetic medium, CD-ROM, DVD-ROM, Blu-Ray ROM, USB thumb drive, SD Card, any other optical medium, punch cards, paper tape, any other physical medium with patterns of holes, RAM, PROM, EPROM, FLASH-EPROM, any other memory chip or cartridge, or any other medium from which a computer may read.

Instructions may further be transmitted or received using a transmission medium. The term "transmission medium" may include any tangible or intangible medium that is capable of storing, encoding or carrying instructions for execution by the machine, and includes digital or analog communications signals or other intangible medium to facilitate communication of such instructions. Transmission media includes coaxial cables, copper wire, and fiber optics, including wires that comprise bus 202 for transmitting a computer data signal. In some examples, execution of the sequences of instructions may be performed by a single computer system 200. According to some examples, two or more computer systems 200 coupled by communication link 220 (e.g., LAN, Ethernet, PSTN, or wireless network) may perform the sequence of instructions in coordination with one another. Computer system 200 may transmit and receive messages, data, and instructions, including programs, (i.e., application code), through communication link 220 and communication interface 212. Received program code may be executed by processor 204 as it is received, and/or stored in disk drive 210, or other non-volatile storage for later execution. Computer system 200 may optionally include a wireless transceiver 213 in communication with the communication interface 212 and coupled 215 with an antenna 217 for receiving and generating RF signals 221 , such as from a WiFi network, BT radio, or other wireless network and/or wireless devices, for example. Examples of wireless devices include but are not limited to: a data capable strap band, wristband, wristwatch, digital watch, or wireless activity monitoring and reporting device; a smartphone; cellular phone; tablet; tablet computer; pad device (e.g., an iPad); touch screen device; touch screen computer; laptop computer; personal computer; server; personal digital assistant (PDA); portable gaming device; a mobile electronic device; and a wireless media device, just to name a few.

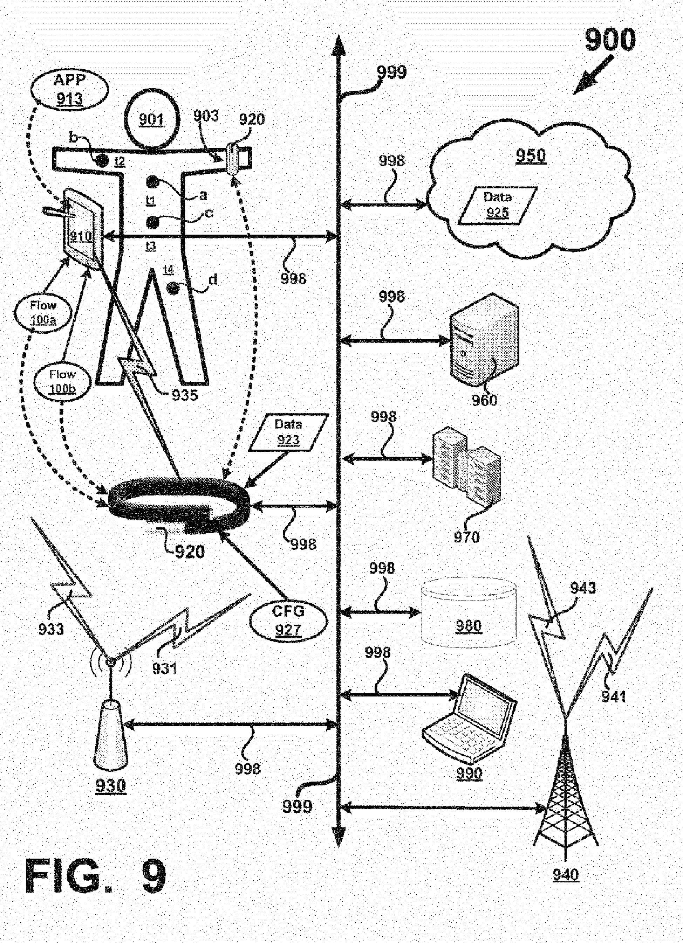

FIG. 9 depicts one example of a system 900 including two wirelessly enabled user devices 910 and 920 for estimating body fat in a user 901. System 900 includes a network 999 and network communications links 988 between the network 999 and various other systems in communications with the network 999. Network communications links 988 may be a wired link (e.g., LAN, Ethernet, USB, FireWire, Thunderbolt, Lightning, etc.) a wireless link (e.g., Bluetooth, WiFi, WiMAX, Cellular, etc.), or both. System that may be in communication 998 with the network 999 and with one another include but are not limited to: wireless user device 910 (e.g., the electronic device of FIGS. 1A - 1B and 3 - 4C); wireless device 920 (e.g., a data capable strap band, wristband, wristwatch, digital watch, or wireless activity monitoring and reporting device); wireless network 930; cellular system 940; resource 950 (e.g., Internet, Cloud, social or processional network, web site, web page, etc.); server 960; data center 970; data storage system 980 (e.g., NAS, RAID, SSD, HDD, Flash memory, etc.); and laptop computer or other form of compute engine (e.g., a PC). Wireless network 930 may be a WiFi router and may send 931 and receive 933 wireless communications to/from the other systems depicted in FIG. 9 including but not limited to wireless devices 910 and 920, for example. Cellular system 940 may be a cellular network and may send 941 and receive 943 wireless communications to/from the other systems depicted in FIG. 9 including but not limited to wireless devices 910 and 920, for example.

In FIG. 9, user 901 has a personal electronics device 910 that may include an application APP 913 executing on the device 910 that provides for estimating body fat of user 901 using device 910 and optionally at least another device, such as wireless device 920. Here, APP or other algorithm embodied in a non-transitory computer readable medium, executing on device 910, instructs user 901 as described above, to position device 910 at one or more various points denoted as a - d on the body of user 901 and to manually apply or apply using hardware in device 910 one or more impulses denoted as t1 - t4, at instructed points on the user's body. APP 913 may include algorithms for executing flow 100a, flow 100b, or both. Alternatively, one or both of the flow (100a, 100b) may be separate algorithms installed on device 910. Device 910 may process inputs and outputs of the flows internally, externally, or both. External processing, including transmitting any data needed for the external processing may be over and network communications links 988 to any of the other systems depicted in FIG. 9, such as user device 920, server 960, data center 970, resource 950, and laptop 990, for example.

User device 920 may be worn, carried, or otherwise connected with user 901 on a portion of the user's body, such as proximate 903 a wrist of the user 901. User device 920 may include data 923 gathered by one or more sensors and/or systems of device 920. Portions of data 923 may have been entered by user 901 using a device such as 910, 990, or an input device such as a keyboard (e.g., a Bluetooth keyboard), for example. Data 923 may be accessed by user device 910 using any of the network communications links 998. Furthermore, user device may directly access data 923 using a wireless link 935 between device 910 and device 920. Data 923 may comprise information about user 901 including but not limited to age, gender, medical history, medication, diet, diet plan, water consumption, exercise regimen, average activity level, physical body dimension, physical condition, body weight, sleep patterns, calorie intake, calories burned, stress levels, heart rate, body temperature, and biometric data, just to name a few.

User device 901 may use at least a portion of data 923 in its analysis for estimating body fat of user 901. Percent body fat and other data presented to user 901 (e.g., in FIG. 4A) may be computed using at least a portion of data 923. Other data 925 that is different than data 223, is similar to 923, is a subset or superset of 923, or is identical to 923 may reside external to device 920 and 910. For example, other data 925 may reside in resource 950; however, it may also reside in one or more other systems such as 960, 970, 980, or 990 where the other data 925 is accessed by 910 and/or 920 using one or more of the network communications links 998. Results from the estimating the body fat of user 901 may subsequently, in part or whole be stored in a data storage medium (e.g., Flash memory) in device 910, 920, or both, or may be transmitted over the network communications links 998 to resource 950, or other systems such as 960, 970, 980, or 990.

User device 920 may include one or more systems operative to implement some or all of the steps of flow 110a and/or 110b. In an alternative example, user device 920 includes one or more of an accelerometer, a gyroscope, or vibration engine. In this example, user device 910 may display information for user 901 on its display in a manner similar to that depicted in FIGS. 3 - 4C; however, the user device 920 is positioned at one or more points on user 901 's body (e.g., a - d) according to the instructions presented on a display of device 910 and device 920 using its internal systems such as the accelerometer and/or gyroscope, sense the vibrations cause by the user 901 manually applying the impulses (e.g., using a hand) at the instructed locations (e.g., t1 -t4). Alternatively, user device 920 may use its vibration engine to generate the impulses (e.g., by a wireless impulse generation command from user device 910). Data from sensors such as the accelerometer and/or gyroscope in user device 920 may be processed internally using flow 110a and/or 110b, for example, and then results from the processing may be wirelessly communicated (e.g., 935) from user device 920 to user device 910. Data from sensors such as the accelerometer and/or gyroscope in user device 920 may be wirelessly communicated (e.g., 935) to user device 910 and processed in user device 910 using flow 110a and/or 110b, for example. Results from the processing (e.g., in 910 or in 920) may then be displayed on a display of user device 910 or be wirelessly communicated using one or more of the network communications links 998 to some other system in communication with the using one or more of the network communications links 998. A configuration file CFG 927 or other data structure in user device 920 may be configured to allow for user device 920 to work in cooperation with user device 910 (e.g., using APP 913) for estimating body fat of user 901. User device 920 and/or 910 may include a port, such as a USB, mini-USB, mini-jack, or other that allows the device to be connected with another system for uploading and/or downloading of data, and may optionally be used for supplying electrical power and/or recharging a battery in those devices.

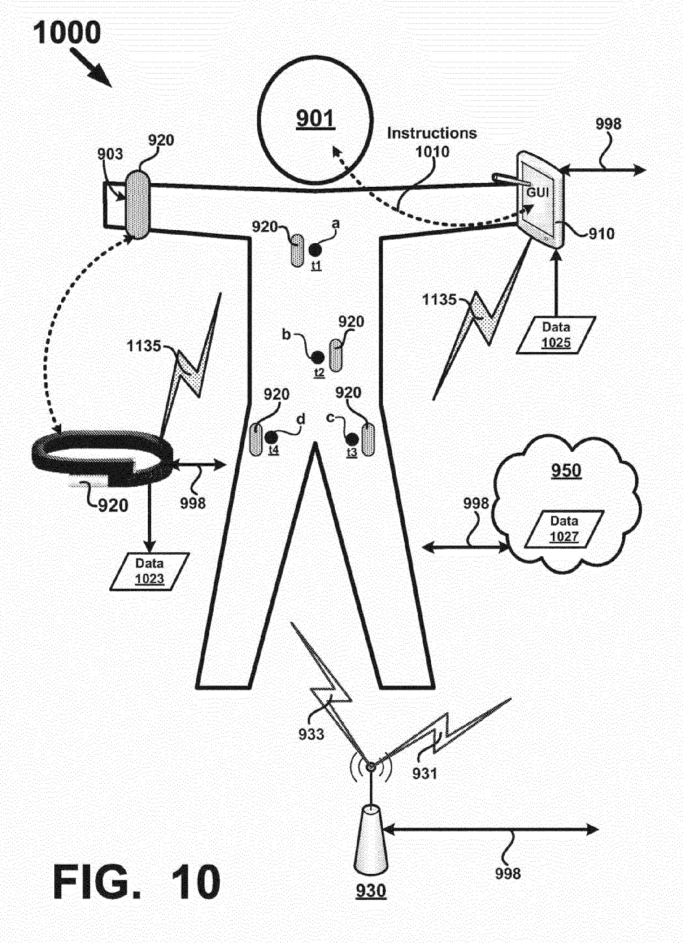

Turning now to FIG. 10, a system 1000 includes at least two user devices denoted as 910 and 920 as described above in reference to FIG. 9. Devices 910 and 920 may be in wireless communications 1135 with each other and may be in communications with the network 999 (not shown) via network communications links 998 as depicted in FIG. 9. Furthermore, Devices 910 and 920 may be in wireless communications with wireless network 930 (e.g., a WiFi network). In FIG. 10, user 901 may remove user device 920 (e.g., a data capable strap band, wristband, wristwatch, digital watch, or wireless activity monitoring and reporting device) from its normal wearing location of position (e.g., proximate wrist 903) and per instructions 1010 displayed (e.g., via a GUI) on a display or screen of device 910, position device 920 at one or more instructed positions on user 901 body as denoted by positions a - d. The user 901 may be instructed to apply impulses at one or more points on the body as denoted by t1 - t4 using a part of the user's body such as a hand or one or more fingers. Alternatively, the user device 920 may apply the impulses to the one or more point's t1 -t4 using one of its systems such as a vibration engine or the like. User device 910 may wirelessly 1135 command the user device 920 to apply an impulse. For example, the GUI may include an icon "Apply Impulse" that the user 901 may activate by pressing the icon with a finger or a stylus. User device 920 may be recording or otherwise collecting data 1023 from the body of user 901 that is related to estimating the body fat of user 901 as described above. That data may reside in a data storage system of user device 920 (e.g., Flash memory) and/or at least a portion of the data 1023 may be wirelessly transmitted 1135 to user device 910 were it is denoted as data 1025 or to some other system such as resource 950 (e.g., data 1027) via network communications links 998 as depicted in FIG. 9. Processing of data (1023, 1025) may occur internal to the devices in which the data resides or external to those devices as described above in reference to FIG. 9. Wireless communications 1135 between user device 920 and 910 may be accomplished using one or more wireless protocols such as Bluetooth and/or WiFi, for example. In some examples, user device 920 may be unstrapped, unfolded, unrolled, or otherwise manipulated or structurally re-configured to perform the functions necessary for estimating the body fat of user 901. For example, one or more specific portions of the user device 920 may include the necessary sensors (e.g., accelerometer, gyroscope, or other) or vibration engine, and the user device 920 may need to be structurally reconfigured so that those portions may be positioned in contact with the body of user 901 to effectuate accurate estimations of the body fat of user 901.

The exemplary computer system 200 depicted in FIG. 2 may be used for systems such as 960, 970, and 990, and may also be used for user device 910 and/or user device 920. User devices 910 and/or 920 may include one or more processors, including multiple core processors, such as a µP, a µC, a DSP, a FPGA, a baseband processor, or ASIC for handling processing chores for flows 110a and/or 110b and other control and processing functions for devices 910 and 920.

The systems, apparatus and methods of the foregoing examples may be embodied and/or implemented at least in part as a machine configured to receive a non-transitory computer-readable medium storing computer-readable instructions. The instructions are preferably executed by computer-executable components preferably integrated with the application, server, network, website, web browser, hardware/firmware/software elements of a user computer or electronic device, or any suitable combination thereof. Other systems and methods of the preferred embodiment may be embodied and/or implemented at least in part as a machine configured to receive a non-transitory computer-readable medium storing computer-readable instructions. The instructions are preferably executed by computer-executable components preferably integrated by computer-executable components preferably integrated with apparatuses and networks of the type described above. The non-transitory computer-readable medium may be stored on any suitable computer readable media such as RAMs, ROMs, flash memory, EEPROMs, optical devices (CD, DVD or Blu-Ray), hard drives, floppy drives, or any suitable device. The computer-executable component may preferably be a processor but any suitable dedicated hardware device may (alternatively or additionally) execute the instructions.

As a person skilled in the art will recognize from the previous detailed description and from the drawing FIGS, and claims set forth below, modifications and changes may be made to the preferred embodiments of the present application without departing from the scope of this present application as defined in the following claims.

Although the foregoing examples have been described in some detail for purposes of clarity of understanding, the above-described inventive techniques are not limited to the details provided. There are many alternative ways of implementing the above-described invention techniques. The disclosed examples are illustrative and not restrictive.

Aspects of the disclosure herein may be summarised in the following clauses:
1. A method for estimating body fat, comprising: providing a user device configured for one or more functions not associated with estimating body fat, the user device including program instructions fixed in a non-transitory computer readable medium, and the program instructions execute on a processor of the user device and are operative to re-configure the user device for estimating body fat; instructing a user on placement of the user device on one or more positions on a body; recording through the user device, an impulse response of the body to an impact applied to the body; and estimating a body fat value of the body by analyzing the impulse response.
2. The method of clause 1, wherein the body is the user's body.
3. The method of clause 1, wherein the instructing comprises the user visually perceiving instructions displayed by the user device.
4. The method of clause 1, wherein the impact is applied by a system of the user device.
5. The method of clause 1, wherein the impact is applied by the user.
6. The method of clause 1, wherein the user device is a device selected from the group consisting of a smartphone, a cellular phone, a tablet, a tablet computer, a pad device, a touch screen device, a touch screen computer, a laptop computer, a personal computer (PC), a server, a personal digital assistant (PDA), a portable gaming device; a mobile electronic device, and a wireless media device.
7. The method of clause 1 and further comprising: displaying the body fat value of the body on a display of the user device.
8. The method of clause 1, wherein the instructing further comprises instructing the user where, when and at what magnitude to impact the body.
9. The method of clause 1, wherein the estimating further comprises estimating a physical dimension of the body.
10. The method of clause 9, wherein the estimating the physical dimension of the body comprises using the user device to do the estimating of the physical dimension of the body.
11. The method of clause 10, wherein the estimating the physical dimension of the body comprises estimating a body fat distribution of the body.
12. The method of clause 11 and further comprising: generating a health-related recommendation for the user.
13. The method of clause 9 and further comprising: generating a health-related recommendation for the user.
14. The method of clause 9, wherein the analyzing further comprises matching the impulse response to a template impulse response.
15. The method of clause 9, wherein the analyzing further comprises correlating time-domain impulse response to a body fat value.
16. The method of clause 9, wherein the analyzing further comprises correlating frequency-domain impulse response to a body fat value.

## Claims

1. A device configured for a primary function and re-configured for estimating body fat of a user, the device comprising:
a user device including a processor;
program instructions fixed in a non-transitory computer readable medium and configured to execute on the processor, the program instructions operative to re-configure the user device for estimating body fat;
a vibration engine included in the user device and operative to generate an impulse;
a display included in the user device and operative to display instructions including where to position the user device on a body and when and at what magnitude to impact the body with the impulse from the vibration engine; and
an accelerometer include in the user device and configured to sense acceleration along one or more axes, the accelerometer operative to generate one or more signals that are analyzed by the processor using the program instructions to estimate the body fat of the body.

2. The device of Claim 1 and further comprising:
a gyroscope included in the user device and configured to sense rotational acceleration, rotational velocity, or both about one or more axes, the gyroscope operative to generate one or more signals that are analyzed by the processor using the program instructions to estimate the body fat of the body.

3. The device of Claim 2, wherein the one or more signals are processed by the processor to implement inertial navigation to guide placement of the electronic device on one or more portions of the body.

4. The device of Claim 2, wherein the one or more signals from the accelerometer, the gyroscope, or both are generated in response to sensing the impulse from the vibration engine.

5. The device of Claim 1, wherein the program instructions are further operative to generate a health-related recommendation.
